# EUROPÄISCHE PATENTSCHRIFT

(11) **EP 0 761 187 B1**
(45) Veröffentlichungstag und Bekanntmachung des Hinweises auf die Patenterteilung: **03.05.2000**
(21) Anmeldenummer: 96112202.5
(22) Anmeldetag: 29.07.1996
(51) Int. Cl.: A61F 13/00

(54) **Trägermaterial für medizinische Zwecke**
Carrier material for medical uses
Matérial de support à usage médical

(30) Priorität: 25.08.1995 DE 19531291
(43) Veröffentlichungstag der Anmeldung: 12.03.1997
(73) Patentinhaber: Beiersdorf Aktiengesellschaft, 20245 Hamburg (DE)
(72) Erfinder: Bodenschatz, Stefan, Dr., 21614 Buxtehude (DE); Himmelsbach, Peter, 21614 Buxtehude (DE)

(56) Entgegenhaltungen:
- EP-A- 0 341 875
- AU-A- 7 355 574
- BE-A- 821 734
- DE-C- 571 244
- DE-C- 619 710
- GB-A- 2 132 939

## Beschreibung

Die Erfindung betrifft ein Trägermaterial für medizinische Zwecke, vorzugsweise Binden, Bandagen, Pflaster oder Wundverbände.

Als Trägermaterialien für diese Zwecke sind bereits zahlreiche Materialien auf Folien-, Gewebe, Gewirke, Vlies-, Gel- oder Schaumstoff-Basis bekannt und werden auch in der Praxis eingesetzt. Die Materialien, welche oft noch mit einer Selbstklebemasse beschichtet sind, müssen hautverträglich, in der Regel luft- und wasserdampfdurchlässig sowie gut anmodellierbar und anschmiegsam sein. Aufgrund dieser Anforderungen wird häufig ein möglichst dünner oder weicher Träger bevorzugt. Zur Handhabung und beim Gebrauch ist bei den Trägermaterialien aber auch eine ausreichende Festigkeit und gegebenenfalls begrenzte Dehnbarkeit gefordert.

Dünne Träger, insbesondere aus Vliesen, sind gut luft- und wasserdampfdurchlässig. Für bestimmte Anwendungen ist jedoch ihre Festigkeit zu gering. Folien sind aufgrund ihrer Flexibilität sehr gut anmodellierbar. Negativ wirkt sich die Dehnbarkeit oft auf die Applikation aus, so daß ihre Einsatzfähigkeit ebenfalls begrenzt ist.

Für spezielle Anwendungen, z.B. Tapes für funktionelle Tapeverbände zur Prophylaxe und Therapie von Verletzungen, Erkrankungen und Veränderungen am Bewegungsapparat, sind unelastische Träger mit einer hohen Festigkeit in Beanspruchungsrichtung erforderlich. Dies wird erreicht, indem Gewebe, üblicherweise aus Baumwolle oder Viskose, eingesetzt werden. In der Regel sind solche Trägermaterialien, mit entsprechend hohem Flächengewicht, kostenintensiv. Eine hohe Flexibilität ist nur durch ein Gewebe mit geringerer Festigkeit zu erreichen. Dieses weist im allgemeinen aber dann unter Beanspruchung eine gewisse Dehnung auf, welche für die Anwendung unerwünscht ist.

Eine weitere Anwendung sind Rollenpflaster und Wundschnellverbände. In der Regel ist es auch hier erforderlich, daß der Träger eine ausreichende Festigkeit aufweist. Die minimale Dicke der Träger ist daher durch die nötige mechanische Festigkeit der Träger begrenzt und um eine Mindestfestigkeit zu haben, ist die Dicke nicht beliebig reduzierbar.

In der AU A 73 55 574 wird offenbart u.a. ein klebendes Produkt, das aus zumindest zwei Trägerschichten besteht, von denen eine als Polyurethanschaumschicht ausgeführt ist. Zwischen den beiden Schichten ist eine klebende Beschichtung vorhanden. Zwischen den beiden äußeren Schichten können verstärkende Fäden eingelegt sein, wobei diese aber nicht in den äußeren Schichten, sondern in der Klebmasse angeordnet sind.
Die Verstärkungen werden nicht in die tragenden Schichten eingearbeitet.

Die GB A 2 132 939 beschreibt ein Laminat aus zwei Schichten, und zwar bestehend aus einem schmelzgeblasenen Träger aus Mikrofasern, auf dem ein verstärkendes Vlies auflaminiert wird, wobei einzelne regionale Verknüpfungspunkte zwischen dem Träger und dem Vlies vorhanden sind.

Die DE C 619 710 offenbart die Verwendung von Verstärkungsfäden, allerdings ist als Trägermaterial, in das die Verstärkungsfäden eingelagert sind, Papier zwingend vorgeschrieben. Des weiteren sind die Verstärkungsfäden nicht charakterisiert.

Die gleichen Merkmale wie die Papierbinde aus DE C 619 710, weist auch die Papierbinde aus DE C 571 244 auf.

Die BE A 821 734 beschreibt kein medizinisches Trägermaterial, sondern ein Klebeband, ohne daß die eingesetzten Verstärkungen charakterisiert werden.

Aufgabe der Erfindung war es deshalb, ein Trägermaterial zu entwickeln, welches die Mindest- oder auch die Optimalanforderungen an Zugfestigkeit und Reißdehnung erfüllt, obwohl der Träger an sich dies nicht tut.

Gelöst wird diese Aufgabe, indem den Trägermaterialien hochfeste Fasern oder Fäden mit einer Höchstzugkraft von mindestens 60 cN/tex zugesetzt werden, welche dem Material eine Höchstzugkraft von mindestens 2 N/cm verleihen gemäß den Merkmalen der unabhängigen Ansprüche 1 bis 3. Die Höchstzugkraftdehnung (Reißdehnung) der erfindungsgemäßen Träger ist dann vorzugsweise unter 400%.

Dadurch werden Trägermaterialien für medizinische Zwecke nutzbar, deren Einsatz bisher an mangelnder Festigkeit und/oder zu hoher Dehnung gescheitert ist.

Als Trägermaterialien können die bekannten Träger aus Gewebe, Gewirke, Folien, Vlies, Schaumstoff, Gele oder Verbundprodukte daraus verwendet werden, sofern sie ansonsten die Anforderungen an den medizinischen Einsatz erfüllen.

Die hochfesten Fasern oder Fäden können aus organischen und anorganischen Materialien bestehen, beispielsweise und bevorzugt aus Glas, Kohlenstoff oder auch speziellen Polyamiden.

Die Fäden können dabei als Monifil, Multifil oder Spinnfasergarn und die Fasern beispielsweise in orientierter Form eingesetzt werden. Sie sollten fest mit dem Trägermaterial verbunden sein. Dies kann durch direktes Einarbeiten der Fasern oder Fäden in den Träger geschehen, wie Einweben bei Geweben, Einstricken bei Gewirken, Einbetten bzw. Einfügen beim Herstellungsverfahren von Folien, Gelen oder Schaumstoffen und Vliesen.

Die Fasern oder Fäden können aber auch nachträglich mit dem Träger verbunden werden, beispielsweise durch Auflaminieren mit einer entsprechenden Verbindungsschicht. Das Einlegen in die Klebmassenschicht bietet sich hierzu zum Beispiel an.

Die Anzahl der an- oder eingebrachten Fäden bzw. Fasern hängt in erster Linie vom jeweils vorgesehenen Verwendungszweck und der angestrebten Höchstzugkraft sowie Höchstzugkraftdehnung des Trägermaterials, seiner eigenen Beschaffenheit und der jeweiligen Festigkeit der Fasern und Fäden selbst ab und kann deshalb in relativ weiten Grenzen variieren. Sie werden vorzugsweise gezielt entsprechend der Beanspruchungsrichtung des Trägermaterials eingefügt, d.h. in Längsrichtung. Sie können jedoch auch, wenn dies zweckdienlicher ist, zusätzlich oder nur in Quer- oder Schrägrichtung oder beispielsweise kurven-, spiral- oder zick-zackförmig oder regellos verlaufen. Dabei kann es wünschenswert und erreichbar sein, daß das Trägermaterial senkrecht zur Orientierung der Verstärkung und/oder in Richtung der Orientierung von Hand einreißbar ist.

Als Trägermaterial für einen funktionellen Tapeverband kann beispielsweise ein flexibles Trägergewebe aus Baumwolle verwendet werden, dem erfindungsgemäß Kohlenstoff (Carbon)- oder Glasfaserfäden in der Kette zugesetzt sind. So kann jeder 2-10te Kettfaden aus dem hochfesten Material bestehen. Durch diese Konstruktion bleibt der Träger flexibel und läßt sich gut anmodellieren. Er hat dabei eine hohe Reißfestigkeit und seine Dehnbarkeit in Beanspruchungsrichtung ist deutlich verringert. Aufgrund der Sprödigkeit der hochfesten Fäden bleibt das Gewebe von Hand reißbar.

Ein zum Tapen geeigneter derartiger Träger weist beispielsweise eine Höchstzugkraft von mindestens 60 N/cm, vorzugsweise 80 bis 100 N/cm, und eine Höchstzugkraftdehnung unter 25%, vorzugsweise 5 bis 10%, auf bei einem Flächengewicht kleiner als 140 g/m², vorzugsweise kleiner als 100g/m².

Bei einer anderen möglichen Ausführungsform besteht das Gewebe aus 100% hochfesten Materialien in der Kette und ergibt Tapeträger mit besonders hoher Zugkraft und geringer Dehnung.

Durch Einfügen oder Auflaminieren der hochfesten Fasern oder Fäden in Vliese oder Folien können Trägermaterialien hergestellt werden, die sich ebenfalls zum Einsatz als Tape eignen. Bei Fertig-Tapeverbänden werden die Verstärkungsfäden oder -fasern bevorzugt gleich entsprechend der Beanspruchungsrichtung im angelegten Zustand angeordnet. Da diese Verbände schon fertig zugeschnitten oder ausgestanzt sind, ist eine Reißbarkeit hier nicht gefordert.

Während für die Tapeverbände eine gewisse Materialstärke nicht unterschritten werden kann, damit sie den hohen Anforderungen gerecht werden können, lassen sich für den Einsatz als Verbandpflaster (Rollenpflaster) und Wundschnellverbände mit Wundauflage auch deutlich leichtere Materialien, insbesondere auf Basis Vlies oder Folie, verwenden, welche durch den Zusatz der hochfesten Fäden oder Fasern doch noch ausreichend stabil sind.

Als Trägermaterial für Verbandpflaster eignet sich beispielsweise ein Träger auf der Basis von Acetat-Gewebe, welcher durch den Zusatz eine Höchstzugkraft von über 40 N/cm, vorzugsweise 60 bis 80 N/cm, und eine Höchtzugkraftdehnung von unter 80%, vorzugsweise 20 bis 30%, aufweist bei einem Flächengewicht von höchstens 90g/m², vorzugsweise 70 bis 50 g/m².

Durch Laminieren, Einarbeiten oder Einlassen von hochfesten Fasern oder Fäden in Vliese oder Folien können Träger von geringem Flächengewicht unter 70 g/m², vorzugsweise unter 40 g/m², mit ausreichender Festigkeit (Höchstzugkraft mindestens 2N/cm bei einer Höchstzugkraftdehnung von unter 400%) für Wundschnellverbände hergestellt werden

Der Einsatz von hochfesten Verstärkungungsfäden oder -fasern ist insbesondere für Anwendungen geeignet, bei denen mit einem dünnen, flexiblen, kostengünstigen, für medizinische Zwecke vorgesehenen Träger ausreichende Festigkeit mit geringer Dehnbarkeit erreicht werden soll.

Je nach Verwendungszweck werden die verstärkten Trägermaterialien gegebenenfalls mit einer der bekannten hautverträglichen Klebemassen auf Kautschuk- oder synthetischen Polymeren-Basis beschichtet, weiterhin gegebenenfalls mit einer Wundauflage versehen und in üblicherweise konfektioniert.

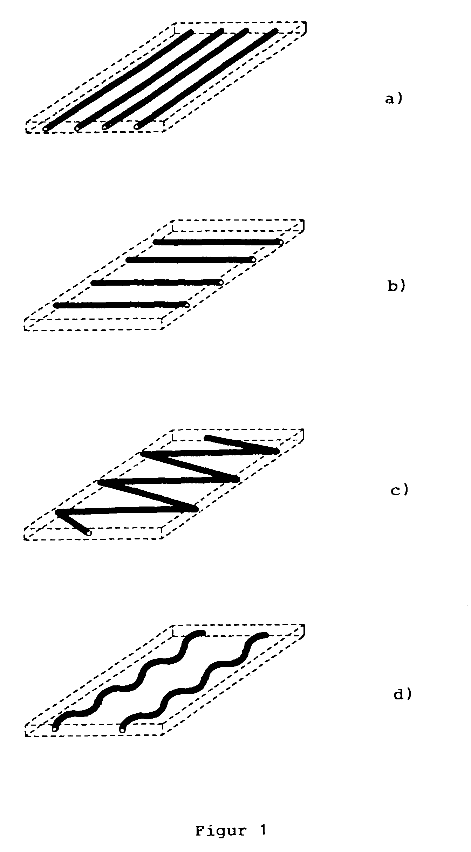

Die Figur 1 a - d zeigt beispielhaft verschiedene Anordnungsmöglichkeiten der Verstärkungsfäden in Längs-, Quer-, Zickzack- und Wellenform.

## Patentansprüche

1. Trägermaterial für medizinische Zwecke, wobei das Trägermaterial aus Vlies, Gewebe, Folie, Schaumstoff, Gel oder Maschenware besteht, dadurch gekennzeichnet, daß ein Zusatz von hochfesten Fasern oder Fäden auf organischer oder anorganischer Basis mit einer Höchstzugkraft über 60 cN/tex dem Trägermaterial eine Höchstzugkraft über 2 N/cm verleiht, wobei das Trägermaterial mit den Fäden und/oder Fasern laminiert ist.

2. Trägermaterial für medizinische Zwecke, wobei das Trägermaterial aus Vlies, Gewebe, Folie, Schaumstoff, Gel oder Maschenware besteht, dadurch gekennzeichnet, daß ein Zusatz von hochfesten Fasern oder Fäden auf organischer oder anorganischer Basis mit einer Höchstzugkraft über 60 cN/tex dem Trägermaterial eine Höchstzugkraft über 2 N/cm verleiht, wobei die Fäden und/oder Fasern in das Trägermaterial eingearbeitet sind.

3. Trägermaterial für medizinische Zwecke, wobei das Trägermaterial aus Vlies, Gewebe, Folie, Schaumstoff, Gel oder Maschenware besteht, dadurch gekennzeichnet, daß ein Zusatz von hochfesten Fasern oder Fäden auf organischer oder anorganischer Basis mit einer Höchstzugkraft über 60 cN/tex dem Trägermaterial eine Höchstzugkraft über 2 N/cm verleiht, wobei die Fäden und/oder Fasern in das Trägermaterial eingelassen sind.

4. Trägermaterial für medizinische Zwecke nach den Ansprüchen 1 bis 3, dadurch gekennzeichnet, daß das Trägermaterial eine Höchstzugkraftdehnung unter 400% aufweist.

5. Trägermaterial für medizinische Zwecke nach den Ansprüchen 1 bis 4, dadurch gekennzeichnet, daß das Trägermaterial mit einem Faden oder mehreren Fäden aus Monofil, Multifil oder Spinnfasergarn verstärkt ist.

6. Trägermaterial für medizinische Zwecke nach den Ansprüchen 1 bis 4, dadurch gekennzeichnet, daß das Trägermaterial mit orientierten Fasern verstärkt ist.

7. Trägermaterial für medizinische Zwecke nach den Ansprüchen 1 bis 6, dadurch gekennzeichnet, daß die Fasern oder Fäden aus Glas, Kohlenstoff oder Polyamiden bestehen.

8. Trägermaterial für medizinische Zwecke, insbesondere für Tape-Bänder, nach einem oder mehreren der vorhergehenden Ansprüche, dadurch gekennzeichnet, daß durch den Zusatz von hochfesten Fasern oder Fäden mit einer Höchstzugkraft über 60 cN/tex das Trägermaterial eine Höchstzugkraft über 60 N/cm und eine Höchstzugkraftdehnung unter 25% bei einem Flächengewicht von unter 140 g/m² hat.

9. Trägermaterial für medizinische Zwecke, insbesondere für Rollenpflaster (Verbandpflaster), nach einem oder mehreren der vorhergehenden Ansprüche, dadurch gekennzeichnet, daß durch den Zusatz von hochfesten Fasern oder Fäden mit einer Höchstzugkraft über 60 cN/tex das Trägermaterial eine Höchstzugkraft über 40 N/cm und eine Höchstzugkraftdehnung unter 80% bei einem Flächengewicht von unter 90 g/m² hat.

10. Trägermaterial für medizinische Zwecke, insbesondere für Wundschnellverbände, nach einem oder mehreren der vorhergehenden Ansprüche, dadurch gekennzeichnet, daß durch den Zusatz von hochfesten Fasern oder Fäden mit einer Höchstzugkraft über 60 cN/tex das Trägermaterial eine Höchstzugkraft über 2 N/cm und eine Höchstzugkraftdehnung unter 400% bei einem Flächengewicht von unter 70 g/m² hat.

11. Trägermaterial für medizinische Zwecke nach einem oder mehreren der vorhergehenden Ansprüche, dadurch gekennzeichnet, daß das Trägermaterial von Hand senkrecht zur Orientierung der Verstärkung und/oder in Richtung der Verstärkung reißbar ist.

12. Trägermaterial für medizinische Zwecke nach einem oder mehreren der vorhergehenden Ansprüche, dadurch gekennzeichnet, daß die Orientierung der Verstärkungsfäden oder -fasern entsprechend der Beanspruchung im Gebrauch ausgerichtet ist.

## Claims

1. Backing material for medical purposes, the backing material comprising nonwoven, woven, film, foam, gel or mesh product, characterized in that an addition of high-strength fibres or filaments based on organic or inorganic compounds and having an ultimate tensile stress strength of more than 60 cN/tex gives the backing material an ultimate tensile stress strength of more than 2 N/cm, the backing material being laminated with the filaments and/or fibres.

2. Backing material for medical purposes, the backing material comprising nonwoven, woven, film, foam, gel or mesh product, characterized in that an addition of high-strength fibres or filaments based on organic or inorganic compounds and having an ultimate tensile stress strength of more than 60 cN/tex gives the backing material an ultimate tensile stress strength of more than 2 N/cm, the filaments and/or fibres being incorporated into the backing material.

3. Backing material for medical purposes, the backing material comprising nonwoven, woven, film, foam, gel or mesh product, characterized in that an addition of high-strength fibres or filaments based on organic or inorganic compounds and having an ultimate tensile stress strength of more than 60 cN/tex gives the backing material an ultimate tensile stress strength of more than 2 N/cm, the filaments and/or fibres being embedded into the backing material.

4. Backing material for medical purposes according to Claims 1 to 3, characterized in that the backing material has an ultimate tensile stress elongation of less than 400%.

5. Backing material for medical purposes according to Claims 1 to 4, characterized in that the backing material is reinforced with one or more filaments of monofil, multifil or spun fibre yarn.

6. Backing material for medical purposes according to Claims 1 to 4, characterized in that the backing material is reinforced with oriented fibres.

7. Backing material for medical purposes according to Claims 1 to 6, characterized in that the fibres or filaments comprise glass, carbon or polyamides.

8. Backing material for medical purposes, especially for tape dressings, according to one or more of the preceding claims, characterized in that owing to the addition of high-strength fibres or filaments with an ultimate tensile stress strength of more than 60 cN/tex the backing material has an ultimate tensile stress strength of more than 60 N/cm and an ultimate tensile stress elongation of less than 25% for a basis weight of less than 140 g/m².

9. Backing material for medical purposes, especially for roll plasters (dressing plasters), according to one or more of the preceding claims, characterized in that owing to the addition of high-strength fibres or filaments with an ultimate tensile stress strength of more than 60 cN/tex the backing material has an ultimate tensile stress strength of more than 40 N/cm and an ultimate tensile stress elongation of less than 80% for a basis weight of less than 90 g/m².

10. Backing material for medical purposes, especially for rapid dressings, according to one or more of the preceding claims, characterized in that owing to the addition of high-strength fibres or filaments with an ultimate tensile stress strength of more than 60 cN/tex the backing material has an ultimate tensile stress strength of more than 2 N/cm and an ultimate tensile stress elongation of less than 400% for a basis weight of less than 70 g/m².

11. Backing material for medical purposes according to one of more of the preceding claims, characterized in that the backing material is tearable by hand perpendicularly to the orientation of the reinforcement and/or in the direction of the reinforcement.

12. Backing material for medical purposes according to one or more of the preceding claims, characterized in that the orientation of the reinforcing filaments or fibres is in accordance with the in-service stress.

## Revendications

1. Matériau de support à des fins médicales, le matériau de support étant composé de voile, tissu, film, mousse, gel ou de tissu maillé, caractérisé en ce qu'une addition de fibres ou de fils très résistants à base organique ou inorganique avec une force de traction maximale de plus de 60 cN/tex confère au matériau de support une force de traction maximale de plus de 2 N/cm, le matériau de support étant stratifié avec les fils et/ou les fibres.

2. Matériau de support à des fins médicales, le matériau de support étant composé de voile, tissu, film, mousse, gel ou tricot, caractérisé en ce qu'une addition de fibres ou de fils très résistants à base organique ou inorganique avec une force de traction maximale de plus de 60 cN/tex confère au matériau de support une force de traction maximale de plus de 2 N/cm, les fils et/ou les fibres étant façonnés dans le matériau de support.

3. Matériau de support à des fins médicales, le matériau de support étant composé de voile, tissu, film, mousse, gel ou tricot, caractérisé en ce qu'une addition de fibres ou de fils très résistants à base organique ou inorganique avec une force de traction maximale de plus de 60 cN/tex confère au matériau de support une force de traction maximale de plus de 2 N/cm, les fils et/ou les fibres étant insérés dans le matériau de support.

4. Matériau de support à des fins médicales suivant les revendications 1 à 3, caractérisé en ce que le matériau de support présente une élongation de moins de 400% à la force de traction maximale.

5. Matériau de support à des fins médicales suivant les revendications 1 à 4, caractérisé en ce que le matériau de support est renforcé avec un fil ou plusieurs fils en monofil, multifil ou filé.

6. Matériau de support à des fins médicales suivant les revendications 1 à 4, caractérisé en ce que le matériau de support est renforcé avec des fibres orientées.

7. Matériau de support à des fins médicales suivant les revendications 1 à 6, caractérisé en ce que les fibres ou les fils sont composés de verre, de carbone ou de polyamides.

8. Matériau de support à des fins médicales, en particulier pour bandages à ruban, suivant l'une ou plusieurs des revendications précédentes, caractérisé en ce que, par l'addition de fibres ou de fils très résistants avec une force de traction maximale de plus de 60 cN/tex, le matériau de support a une force de traction maximale de plus de 60 N/cm et une élongation de moins de 25% à la force de traction maximale pour un grammage de moins de 140 g/m².

9. Matériau de support à des fins médicales, en particulier pour emplâtres en rouleaux (sparadrap), suivant l'une ou plusieurs des revendications précédentes, caractérisé en ce que, par l'addition de fibres ou de fils très résistants avec une force de traction maximale de plus de 60 cN/tex, le matériau de support a une force de traction maximale de plus de 40 N/cm et une élongation de moins de 80% à la force de traction maximale pour un grammage de moins de 90 g/m².

10. Matériau de support à des fins médicales, en particulier pour pansements de premiers soins pour plaie, suivant l'une ou plusieurs des revendications précédentes, caractérisé en ce que, par l'addition de fibres ou de fils très résistants avec une force de traction maximale de plus de 60 cN/tex, le matériau de support a une force de traction maximale de plus de 2 N/cm et une élongation de moins de 400% à la force de traction maximale pour un grammage de moins de 70 g/m².

11. Matériau de support à des fins médicales suivant l'une ou plusieurs des revendications précédentes, caractérisé en ce que le matériau de support peut être déchiré à la main perpendiculairement par rapport à l'orientation du renforcement et/ou dans le sens du renforcement.

12. Matériau de support à des fins médicales suivant l'une ou plusieurs des revendications précédentes, caractérisé en ce que l'orientation des fils ou des fibres de renforcement correspond à la direction de la sollicitation dans l'usage.
